# EUROPEAN PATENT SPECIFICATION

(11) **EP 2 268 290 B1**
(45) Date of publication and mention of the grant of the patent: **30.01.2013**
(21) Application number: 09720080.2
(22) Date of filing: 09.03.2009
(51) Int. Cl.: A61K 31/74, A61P 15/16, A61P 35/00

(54) **STYRENE MALEIC ANHYDRIDE BASED FORMULATION FOR MALE CONTRACEPTION AND PROSTATE CANCER**
FORMULIERUNG AUF STYROLMALEINSÄUREANHYDRID-BASIS FÜR MÄNNLICHE KONTRAZEPTION UND GEGEN PROSTATAKREBS
FORMULATION A BASE DE STYRENE-ANHYDRIDE MALEIQUE DESTINEE A LA CONTRACEPTION MASCULINE ET AU CANCER DE LA PROSTATE

(30) Priority: 11.03.2008 IN DE06182008
(43) Date of publication of application: 05.01.2011
(73) Proprietor: Guha, Sujoy Kumar, New Delhi 110 019 (IN)
(72) Inventor: Guha, Sujoy Kumar, New Delhi 110 019 (IN)
(74) Representative: Hirsch & Associés
(86) International application number: PCT/IN2009/000161
(87) International publication number: WO 2009/113108

(56) References cited:
- US-A- 5 488 075
- US-A1- 2002 076 381
- "Te Rise of Risug", The Scholars' Avenue, 8 October 2009 (2009-10-08), XP002628817, Retrieved from the Internet: URL:http://www.scholarsavenue.org/2009/10/ 08/the-rise-of-risug/ [retrieved on 2011-03-18]

## Description

### Field of the Invention:

The present invention relates to styrene maleic anhydride based formulation for male contraception and prostate cancer.

Particularly, the present invention relates to styrene maleic anhydride based formulation for male contraception and prostate cancer which can be injected in vas deferens lumen.

### Background of the Invention:

Prostate cancer is the most common form of cancer in the male with the incidence of the latent form going beyond 60% in the age group above 70 years. Therefore, modalities for preventing the prostate cancer is need of the time. So far there is no proven preventive drug formulation and method.

Only recently, a drug named finasteride [US patent Nos. 6,090,409 and 5,175,155] has been made available, which has major drawback that it has to be administered at regular intervals.

Further drawback of the drug finasteride is that it has shown very minimal benefits towards prevention of prostate cancer.

Further, drawback of the drug finasteride is that besides being low in prevention efficacy, it has the limitations of high cost and considerable side effects.

Accordingly, yet there is a need of a drug, which can be administered for limited numbers, and still demonstrates greater benefits towards prevention of prostate cancer, and still can be reasonably affordable by common man and still has no or minimal side effects.

Further, if formulation for prevention of prostate cancer can also have additional benefit of being suitable as male contraception, it can serve dual purpose with one dosage form, as countries like India also need safer and effective male contraceptives.

The inventor had earlier developed an injectable male contraceptive which is implanted in the vas deferens of the male [US patent No. 5,488,075], and which consists of styrene maleic anhydride [SMA] and dimethyl sulfoxide [DMSO], herein after referred to as SMA contraceptive [RISUG^{®}], and it is undergoing the commercial batch production and the Phase - III Clinical Trials. It has been found that this SMA contraceptive [RISUG®] destroys the sperms passing in the vas deferens, and the destroyed or broken down sperms flow along the vas deferens and pass through the prostate to the ejaculatory duct and finally get out of the penis. It has also been found that there are interchanges between destroyed sperms and secretory products of the prostate gland and such process continues as the sperms are continually produced, destroyed and flow along the vas deferens, and hence, opportunity of intervention at the level of the prostate gland by means of flowing destroyed sperms opens up.

However, it has been observed that the styrene maleic anhydride [SMA] used in the above-defined SMA contraceptive [RISUG®] has very higher molecular weight varying in the range from about 60000 to about 100000. It has been found that this polymer of such a higher molecular weight is very stable and it remains confined to the region of the vas deferens where it is implanted originally, and such a polymer does not serve the need of assembling the nano particles of the formulation, which could also travel along the vas deferens to the target site in the prostate gland as described herein below.

Accordingly, it has been found that even SMA contraceptive [RISUG®] as such does not serve purpose of preventing the prostate cancer.

### Need of the Invention:

Therefore, there is a need of a formulation, which can be administered in a small quantity and that's too for limited number of times in a life span, and still demonstrates greater benefits towards prevention of prostate cancer and also causing male contraception, and still can be reasonably affordable by common man and still has no or minimal side effects.

### Objects and Aim of the Invention:

Therefore, this is the main object of the present invention to provide a formulation, which can be administered in a small quantity and that's too for limited number of times in a life span, preferably only once or twice in life time, and is capable of demonstrating greater benefits towards prevention of prostate cancer and also causing male contraception, and is still reasonably affordable by common man and at the same time predominantly has no or minimal side effects.

Other objects and aim of the invention will become more apparent from the following description when read in conjunction with the accompanying figures which are incorporated merely to illustrate the present invention and not to limit its scope.

### Brief Description of the Accompanying Figures:-

Figure 1 illustrates Transmission Electron Microscopic Image of the vas deferens fluid in rat treated with formulation of the present invention, wherein liposomes and fragments of sperms are seen.
Figure 2 illustrates Fluorescence Microscopic Image using Nile Red as the fluorescent marker of the vas deferens fluid in rat treated with formulation of the present invention, wherein liposomes and fragments of sperms are seen.
Figure 3 illustrates Fluorescence Microscopic Image using Nile Red as the fluorescent marker of the prostate gland in rat treated with formulation of the present invention, showing the liposomes encapsulating nano particles of high molecular weight SMA which have traveled from the vas deferens and got absorbed into the epithelial zone of the prostate gland.

### Detailed Description and Preferred Embodiments of the Invention:

It is understood from the foregoing description that at present there is no drug formulation which can demonstrate greater benefits towards prevention of prostate cancer and also cause male contraception with preferably one or two administrations in life time and is still reasonably affordable by common man and predominantly does not cause side effects. With aim to fill this gap in the technology by developing such drug formulation, the inventor has surprisingly found that when low molecular weight SMA having molecular weight varying in the range of about 10000 to about 20000 is combined with high molecular weight SMA having molecular weight varying in the range of about 60000 to about 100000, the combination of lower molecular weight SMA and the higher molecular weight SMA surprisingly acquires synergistic property to prevent the prostate cancer and also cause male contraception without requiring its administration at regular intervals and predominantly no or minimal side effects, and still being reasonably affordable by common man. It has been surprisingly found that such SMA formulation after causing male contraception in the vas deferens flows along the vas deferens and gets absorbed into the epithelial zone of the prostate gland, which confirms that such SMA formulation also has greater capability towards prevention of prostate cancer in-addition to causing male contraception.

It is observed that low molecular weight SMA is relatively unstable than the high molecular weight SMA. The inventor of this invention has found that the low molecular weight SMA surprisingly creates cleavage centers within the high molecular weight SMA bulk, and the low molecular weight SMA has also been found to have greater tendency to break down as its molecular weight is lowered. However, the inventor surprisingly observed that if molecular weight of the SMA is lowered further beyond molecular weight of 10000 it gives such a high degradation that even the higher molecular weight SMA mass rapidly disintegrates, and therefore, does not serve the purpose of a sustained drug source in the vas deferens, and hence, cannot be used for causing male contraception and as well for preventing the prostate cancer. It has been further found that when low molecular weight SMA and high molecular weight SMA are combined in a limited weight ratio it surprisingly forms appropriate size of the nano particles of the higher molecular weight SMA in the size varying within the range of about 20 to about 100 nanometer diameter. In this manner, the nano particles of the higher molecular weight SMA which have been surprisingly found to be anti-mutagenic are produced by breakdown of the lower molecular weight SMA which in itself does not demonstrate anti-mutagenic property.

It has been found that injecting SMA formulation comprising higher molecular weight SMA and lower molecular weight SMA is difficult. To avoid this difficulty, the SMA formulation is dissolved in dimethyl sulphoxide [DMSO] for ease of injection. The use of DMSO has been found to serve additional surprising benefit by bringing in the sulfur to which the prostate tissues have greater affinity, and therefore, the uptake of the liposomes containing the high molecular weight SMA into the prostate tissue is greatly facilitated.

Accordingly, the present invention relates to a styrene maleic anhydride based synergistic formulation comprising styrene maleic anhydride [SMA] having lower molecular weight and styrene maleic anhydride [SMA] having higher molecular weight dissolved in DMSO, and the formulation being capable of preventing the prostate cancer as well as causing male contraception even when administered in smaller doses for one or two administrations in the life time and predominantly causing no or minimal side effects, and still being reasonably affordable by common man, and the formulation being capable of traveling along the vas deferens after causing male contraception in the vas deferens to the prostate gland and getting absorbed into the epithelial zone of the prostate gland confirming that the formulation has greater capability towards prevention of prostate cancer in-addition to causing male contraception.

Accordingly, the present invention, in one of the preferred embodiments relates to a styrene maleic anhydride based synergistic formulation for male contraception and prostate cancer comprising SMA having lower molecular weight varying in the range from about 10000 to about 20000 and SMA having higher molecular weight varying in the range from about 60000 to about 100000 which are dissolved in DMSO, and the formulation being capable of preventing the prostate cancer as well as causing male contraception.

As described herein, if molecular weight of the SMA having lower molecular weight is lowered further beyond molecular weight of about 10000 it surprisingly gives such a high degradation that the higher molecular weight SMA mass rapidly disintegrates and does not serve the purpose of a sustained drug source for male contraception as well as for prostate cancer.

Accordingly, in accordance with one of the preferred embodiments of this invention, the SMA having lower molecular weight is mixed with SMA having higher molecular weight in a manner that the amount of SMA having higher molecular weight is higher than the SMA having lower molecular weight, preferably the SMA having lower molecular weight is mixed with SMA having higher molecular weight in a ratio varying in the range from about 1 : 4 to about 1 : 6, that is, because as described herein even if amount of the SMA having lower molecular weight is increased beyond defined limits it also surprisingly gives such a high degradation that the higher molecular weight SMA mass rapidly disintegrates and does not serve the purpose of a sustained drug source for prostate cancer and male contraception.

In accordance with one embodiment of the present invention, the SMA formulation comprises predominantly straight chain SMA, and the chain of the SMA may be longer enough. The reason of selecting the straight chain SMA is to have all maleic anhydride groups to be active for sperm break down function.

In accordance with another preferred embodiment of this invention, about 5% to about 15% of the SMA having higher molecular weight is replaced with styrene maleic acid, which has been surprisingly found to enhance the breakdown of sperms, and thereby, to generate adequate quantity of lipids for liposome formation. In accordance with one of the preferred embodiments of the present invention, the molecular weight of the styrene maleic acid is same as that of the high molecular weight SMA, that is, varying in the range from about 60000 to about 100000.

In accordance with one of the preferred embodiments of this invention, the SMA comprising SMA having lower molecular weight and SMA having higher molecular weight when taken in above-defined ratio is dissolved in DMSO for ease of injection in a preferred ratio of about 1:1.5 [about 1 mg of SMA in about 1.5 µl of DMSO] to about 1:3 in weight by volume [about 1 mg of SMA in about 3 µl of DMSO], preferably of about 1 : 2 in weight by volume [about 1 mg of SMA in about 2 µl of DMSO].

Accordingly, the present invention discloses a novel formulation, which generates a means of assembling lipids released by breaking down of sperms and nano particles of higher molecular weight SMA fragments produced by formulation of present invention, in the vas deferens, on a continual basis with one single intervention or at the maximum of two interventions of implantation of present formulation.

Additionally the invention, discloses such a nano particle drug form which is not lost by absorption into the wall of the vas deferens. Instead the in-vivo assembling of lipids released by breaking down of sperms and nano particles of higher molecular weight SMA fragments produced by formulation of present invention is surprisingly transported along the vas deferens to the prostate after it has served function of achieving male contraception.

Further, the presently disclosed drug formulation has demonstrated such a surprising property that it targets onto the secretory epithelium of the prostate gland, which are known to mutate leading to prostate cancer, and hence, the presently disclosed formulation has surprisingly demonstrated mutagenesis inhibiting tendency for preventing epithelial cell from undergoing mutation and becoming cancerous. This act of "quenching" mutation appears to be associated with cell signaling to other cells, thereby, inhibiting mutation in other cells as well.

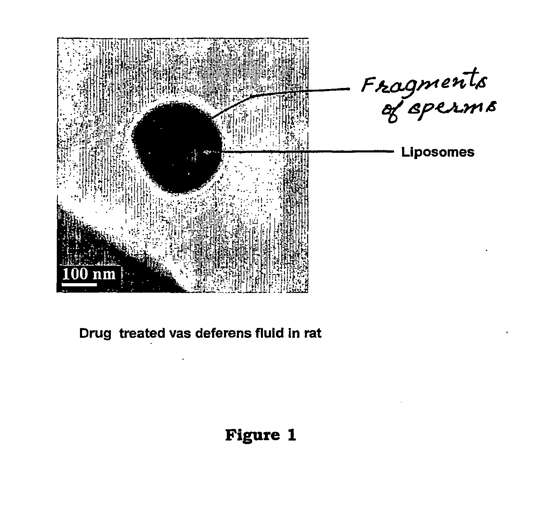

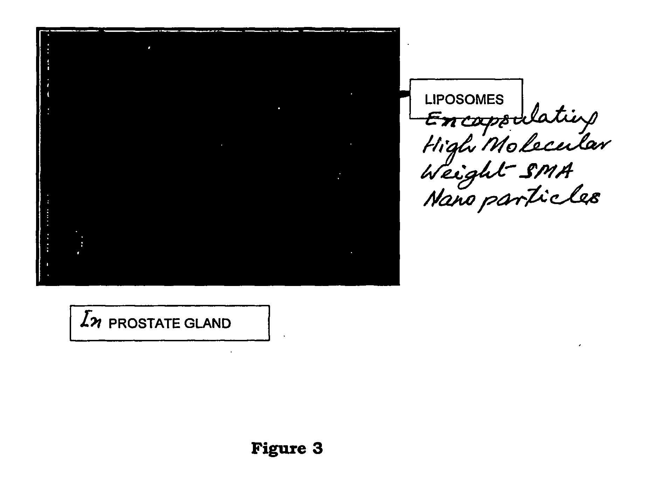

Now referring to the accompanying figures, which are -incorporated merely to illustrate the present invention and not to restrict its scope, wherein Figure 1 illustrates Transmission Electron Microscopic Image taken by Transmission Electron Microscopy of the vas deferens fluid in rat treated with formulation of the present invention, wherein the liposomes containing high molecular weight SMA and also the fragments of sperms can be seen, which goes to confirm that there is break down of high molecular weight SMA to form high molecular weight SMA nano particles which gets encapsulated within the liposomes. In present invention, it has been observed that high molecular weight SMA encapsulated within the liposomes surprisingly travels down the vas deferens and get into the epithelial zone of the prostate tissue as it is illustrated by accompanying Figure 3 illustrating Fluorescence Microscopic Image using Nile Red as the fluorescent marker of the prostate gland in rat treated with formulation of the present invention wherein the liposomes encapsulating the high molecular weight SMA nano particles which have traveled from the vas deferens can be seen.

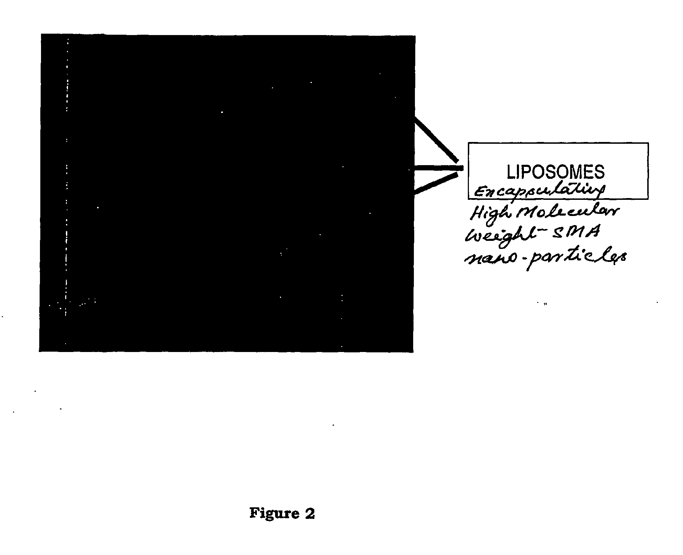

Now referring to the accompanying Figure 2, it illustrates Fluorescence Microscopic Image using Nile Red as the fluorescent marker of the vas deferens fluid in rat treated with formulation of the present invention, wherein the liposomes containing high molecular weight SMA and also the fragments of sperms can be seen, which also goes to confirm that there is break down of high molecular weight SMA to form high molecular weight SMA nano particles which gets encapsulated within the liposomes. It has again been observed that high molecular weight SMA encapsulated within the liposomes travels down the vas deferens and get into the epithelial zone of the prostate tissue as it is illustrated by accompanying Figure 3 illustrating Fluorescence Microscopic Image using Nile Red as the fluorescent marker of the prostate gland in rat treated with formulation of the present invention wherein the liposomes encapsulating the high molecular weight SMA nano particles which have traveled from the vas deferens can be seen.

Further, the in-vitro experimental studies [including AMES studies] conducted on *Salmonella typhimurium* by employing the present formulation have shown that it not only has male contraception property, but also has anti-mutagenic property. By the AMES test, which assays reversion of mutation using *Salmonella typhimurium* as the test strain, it has been tested and observed that with high molecular weight SMA the number of revertants in the SMA treated samples was generally 5 to 20% less than in the controls which clearly indicates that the high molecular weight SMA is capable of demonstrating antimutagenic property that inhibits mutations which are the principal pathways for cancer formation. Therefore, the liposomes with the high molecular weight SMA in the core transferred to the prostate epithelial tissue becomes a cancer inhibiting drug delivered directly to the prostate. This finding leads the conclusion that spontaneous mutations in the prostate will be inhibited by the liposome delivered drug, and thereby, will prevent the initiation of the cancer formation process.

It has been observed that to achieve male contraception, the SMA having higher molecular weight of the present formulation is still capable of demonstrating its pH lowering and electrical charge effects, which is observed to cause breakdown of the sperms. The sperm membrane has proteins and lipids which are released when the sperm breaks down. The spermatic fluid flowing inside the vas deferens has some water. The lipids released from the sperm in the presence of this water forms liposomes since it prevents interaction of water with the hydrocarbon core of the lipid bilayer at the edges. The nano particles of SMA produced in-vivo by present formulation being lipophilic in nature are observed to dissolve within the lipid bilayers. The SMA nano particle is encapsulated within the liposomes formed from the sperm lipid. Since the sperms are continually being formed and flow past the present formulation comprising SMA having higher molecular weight and get break down in transit, there is a continual supply of the lipids. The nano particles each have a small volume and the breakdown rate of the SMA having lower molecular weight is such that the release of the nano particles from SMA having higher molecular weight is slow in the vas deferens region. Thus, one implantation of the present formulation is expected to be a source of drug for prostate cancer for over 15 years or so.

Accordingly, it is understood from the foregoing description that the present invention has provided a formulation capable of being administered in small quantity and that's too for limited number of times in a life span, preferably only once or twice in life time, and capable of being demonstrating greater benefits towards prevention of prostate cancer and also causing male contraception, and still being reasonably affordable by common man and at the same time predominantly causing no or minimal side effects.

## Claims

1. A styrene maleic anhydride based synergistic formulation for preventing prostate cancer and causing male contraception comprising styrene maleic anhydride [SMA] having lower molecular weight and styrene maleic anhydride [SMA] having higher molecular weight dissolved in dimethyl sulfoxide [DMSO], wherein molecular weight of said SMA having lower molecular weight varies in the range from about 10000 to about 20000 and wherein molecular weight of said SMA having higher molecular weight varies in the range from about 60000 to about 100000.

2. A formulation as claimed in claim 1, wherein amount of said SMA having higher molecular weight is higher than said SMA having lower molecular weight.

3. A formulation as claimed in any of claims 1 or 2, wherein said SMA having lower molecular weight is mixed with said SMA having higher molecular weight in a ratio varying in the range from about 1 : 4 to about 1 : 6.

4. A formulation as claimed in any of the preceding claims, wherein said formulation comprises predominantly straight chain SMA.

5. A formulation as claimed in any of the preceding claims, wherein about 5% to about 15% of said SMA having higher molecular weight is replaced with styrene maleic acid.

6. A formulation as claimed in claim 5, wherein molecular weight of styrene maleic acid is same as that of said high molecular weight SMA.

7. A formulation as claimed in any of the preceding claims, wherein said formulation comprising said SMA having lower molecular weight and said SMA having higher molecular weight is dissolved in DMSO in a ratio of about 1:1.5 to about 1:3 in weight by volume.

8. A formulation as claimed in any of the preceding claims, wherein said formulation comprising said SMA having lower molecular weight and said SMA having higher molecular weight is dissolved in DMSO in a ratio of about 1: 2 in weight by volume.

9. A styrene maleic anhydride based synergistic formulation for use in preventing prostate cancer and causing male contraception wherein said formulation comprises styrene maleic anhydride [SMA] having lower molecular weight and styrene maleic anhydride [SMA] having higher molecular weight dissolved in dimethyl sulfoxide [DMSO], wherein molecular weight of said SMA having lower molecular weight varies in the range from about 10000 to about 20000, and wherein the molecular weight of said SMA having higher molecular weight varies in the range from about 60000 to about 100000, and wherein said formulation is administered to travel along the vas deferens, after causing male contraception in the vas deferens, to the prostate gland and to get absorbed into the epithelial zone of the prostate gland for preventing prostate cancer.

10. A formulation as claimed in claim 9, wherein said formulation targets onto the secretory epithelium of the prostate gland and demonstrates mutagenesis inhibiting tendency for preventing epithelial cell from undergoing mutation and becoming cancerous.

11. A formulation as claimed in claims 9 or 10, wherein said formulation quenches mutation associated with cell signaling to other cells and also inhibits mutation in other cells.

12. A formulation as claimed in any of the preceding claims, wherein said high molecular weight SMA breaks down to form high molecular weight SMA nano particles, which get encapsulated within the liposomes.

13. A formulation as claimed in any of the preceding claims, wherein said high molecular weight SMA nano particles encapsulated within the liposomes travel down the vas deferens and get absorbed into the epithelial zone of the prostate tissue for preventing the prostate cancer.

14. A formulation as claimed in any of claims 1 to 8, wherein said high molecular weight SMA breaks down to form high molecular weight SMA nano particles.

15. A formulation as claimed in claim 14, wherein said high molecular weight SMA nano particles get encapsulated within the liposomes, and said high molecular weight SMA nano particles encapsulated within the liposomes travel down the vas deferens and get into the epithelial zone of the prostate tissue.

## Patentansprüche

1. Styrol-Maleinsäureanhydrid-basierte synergistische Formulierung zum Verhindern von Prostatakrebs und zum Bewirken männlicher Verhütung, umfassend Styrol-Maleinsäureanhydrid [SMA] mit niedrigerem Molekulargewicht und Styrol-Maleinsäureanhydrid [SMA] mit höherem Molekulargewicht, gelöst in Dimethylsulfoxid [DMSO], wobei das Molekulargewicht des SMA mit niedrigerem Molekulargewicht im Bereich von etwa 10000 bis etwa 20000 variiert und wobei das Molekulargewicht des SMA mit höherem Molekulargewicht im Bereich von etwa 60000 bis etwa 100000 variiert.

2. Formulierung nach Anspruch 1, wobei die Menge des SMA mit höherem Molekulargewicht höher ist als das SMA mit niedrigerem Molekulargewicht.

3. Formulierung nach einem der Ansprüche 1 oder 2, wobei das SMA mit niedrigerem Molekulargewicht in einem im Bereich von etwa 1:4 bis etwa 1:6 variierenden Verhältnis mit dem SMA mit höherem Molekulargewicht gemischt ist.

4. Formulierung nach einem der vorherigen Ansprüche, wobei die Formulierung vorwiegend lineares SMA umfasst.

5. Formulierung nach einem der vorherigen Ansprüche, wobei etwa 5 % bis etwa 15 % des SMA mit höherem Molekulargewicht durch Styrol-Maleinsäure ersetzt ist.

6. Formulierung nach Anspruch 5, wobei das Molekulargewicht der Styrol-Maleinsäure gleich ist wie das des hochmolekularen SMA.

7. Formulierung nach einem der vorherigen Ansprüche, wobei die Formulierung, welche das SMA mit niedrigerem Molekulargewicht und das SMA mit höherem Molekulargewicht umfasst, in einem Verhältnis von etwa 1:1,5 bis etwa 1:3 nach Volumengewicht in DMSO gelöst ist.

8. Formulierung nach einem der vorherigen Ansprüche, wobei die Formulierung, welche das SMA mit niedrigerem Molekulargewicht und das SMA mit höherem Molekulargewicht umfasst, in einem Verhältnis von etwa 1:2 nach Volumengewicht in DMSO gelöst ist.

9. Styrol-Maleinsäureanhydrid-basierte synergistische Formulierung zur Verwendung beim Verhindern von Prostatakrebs und zum Bewirken männlicher Empfängnisverhütung, wobei die Formulierung Styrol-Maleinsäureanhydrid [SMA] mit niedrigerem Molekulargewicht und Styrol-Maleinsäureanhydrid [SMA] mit höherem Molekulargewicht, gelöst in Dimethylsulfoxid [DMSO], umfasst, wobei das Molekulargewicht des SMA mit niedrigerem Molekulargewicht im Bereich von etwa 10000 bis etwa 20000 variiert und wobei das Molekulargewicht des SMA mit höherem Molekulargewicht im Bereich von etwa 60000 bis etwa 100000 variiert und wobei die Formulierung verabreicht wird, um entlang des Samenleiters, nachdem es im Samenleiter männliche Verhütung bewirkt hat, zur Prostatadrüse zu wandern und in der Epithelialzone der Prostatadrüse zur Verhinderung von Prostatakrebs absorbiert zu werden.

10. Formulierung nach Anspruch 9, wobei die Formulierung auf das Sekretionsepithel der Prostatadrüse abzielt und Mutagenese hemmende Tendenz zeigt, um zu verhindern, dass Epithelzellen eine Mutation durchlaufen und kanzerös werden.

11. Formulierung nach Anspruch 9 oder 10, wobei die Formulierung Mutation in Verbindung mit Zellsignalisierung an andere Zellen dämpft und auch Mutation in anderen Zellen hemmt.

12. Formulierung nach einem der vorherigen Ansprüche, wobei das hochmolekulare SMA zu hochmolekularen SMA-Nanopartikeln zerfällt, die in den Liposomen eingekapselt werden.

13. Formulierung nach einem der vorherigen Ansprüche, wobei die in den Liposomen eingekapselten hochmolekularen SMA-Nanopartikel den Samenleiter entlang wandern und in die Epithelialzone des Prostatagewebes absorbiert werden, um den Prostatakrebs zu verhindern.

14. Formulierung nach einem der Ansprüche 1 bis 8, wobei das hochmolekulare SMA zu hochmolekularen SMA-Nanopartikeln zerfällt.

15. Formulierung nach Anspruch 14, wobei die hochmolekularen SMA-Nanopartikel in den Liposomen eingekapselt werden, und die in den Liposomen eingekapselten hochmolekularen SMA-Nanopartikel den Samenleiter entlang wandern und in die Epithelialzone des Prostatagewebes gelangen.

## Revendications

1. Une formulation synergique à base de styrène anhydride maléique pour prévenir le cancer de la prostate et provoquer une contraception masculine, comprenant du styrène anhydride maléique [SMA] présentant une masse moléculaire plus basse et du styrène anhydride maléique [SMA] présentant une masse moléculaire plus élevée, dissous dans du diméthyl sulfoxyde [DMSO], formulation dans laquelle la masse moléculaire dudit SMA présentant une masse moléculaire plus basse est comprise dans une fourchette d'environ 10 000 à environ 20 000 et dans laquelle la masse moléculaire dudit SMA présentant une masse moléculaire plus élevée est comprise dans une fourchette d'environ 60 000 à environ 100 000.

2. Une formulation telle que revendiquée dans la revendication 1, dans laquelle la quantité dudit SMA présentant une masse moléculaire plus élevée est supérieure à celle dudit SMA présentant une masse moléculaire plus basse.

3. Une formulation telle que revendiquée dans la revendication 1 ou 2, dans laquelle ledit SMA présentant une masse moléculaire plus basse est mélangée avec ledit SMA présentant une masse moléculaire plus élevée dans un rapport compris dans une fourchette d'environ 1/4 à environ 1/6.

4. Une formulation telle que revendiquée dans une quelconque des revendications précédentes, dans laquelle ladite formulation comprend de façon prédominante des chaines linéaires de SMA.

5. Une formulation telle que revendiquée dans une quelconque des revendications précédentes, dans laquelle d'environ 5% à environ 15% dudit SMA présentant une masse moléculaire plus élevée sont remplacés par du styrène/acide maléique.

6. Une formulation telle que revendiquée dans la revendication 5, dans laquelle la masse moléculaire du styrène/acide maléique est identique à celle dudit SMA de masse moléculaire élevée.

7. Une formulation telle que revendiquée dans une quelconque des revendications précédentes, dans laquelle ladite formulation comprenant ledit SMA présentant une masse moléculaire plus basse et ledit SMA présentant une masse moléculaire plus élevée est dissoute dans du DMSO en un rapport d'environ 1/1,5 à environ 1/3 en poids par volume.

8. Une formulation telle que revendiquée dans une quelconque des revendications précédentes, dans laquelle ladite formulation comprenant ledit SMA présentant une masse moléculaire plus basse et ledit SMA présentant une masse moléculaire plus élevée est dissoute dans du DMSO dans un rapport d'environ 1/2 en poids par volume.

9. Une formulation synergique à base de styrène anhydride maléique pour son utilisation pour prévenir le cancer de la prostate et provoquer une contraception masculine, ladite formulation comprenant du styrène anhydride maléique [SMA] présentant une masse moléculaire plus basse et du styrène anhydride maléique [SMA] présentant une masse moléculaire plus élevée dissous dans du diméthyl sulfoxyde [DMSO], dans laquelle formulation la masse moléculaire dudit SMA présentant une masse moléculaire plus basse est comprise dans une fourchette d'environ 10 000 à environ 20 000 et dans laquelle la masse moléculaire dudit SMA présentant une masse moléculaire plus élevée est comprise dans une fourchette d'environ 60 000 à environ 100000, et dans laquelle ladite formulation est administrée en vue qu'elle se déplace dans le canal déférent, après avoir provoqué une contraception masculine dans le canal déférent, atteindre la prostate puis être absorbée dans la zone épithéliale de la prostate afin de prévenir le cancer de la prostate.

10. Une formulation telle que revendiquée dans la revendication 9, dans laquelle ladite formulation cible l'épithélium secrétoire de la prostate et produit une tendance inhibitrice de la mutagénèse pour empêcher la cellule épithéliale de subir une mutation et devenir cancéreuse.

11. Une formulation telle que revendiquée dans les revendications 9 ou 10, dans laquelle ladite formulation bloque la mutation associée au signal émis par une cellule vers d'autres cellules et également inhibe la mutation dans les autres cellules.

12. Une formulation telle que revendiquée dans une quelconque des revendications précédentes, dans laquelle ledit SMA de masse moléculaire élevée se brise pour former des nanoparticules de SMA de masse moléculaire élevée lesquelles s'encapsulent dans des liposomes.

13. Une formulation telle que revendiquée dans une quelconque des revendications précédentes, dans laquelle les nanoparticules de SMA de masse moléculaire élevée encapsulées dans des liposomes se déplacent dans le canal déférent et sont absorbées par la zone épithéliale du tissu prostatique afin de prévenir le cancer de la prostate.

14. Une formulation telle que revendiquée dans une quelconque des revendications 1 à 8, dans laquelle ledit SMA de masse moléculaire élevée se brise pour former des nanoparticules de SMA de masse moléculaire élevée.

15. Une formulation telle que revendiquée dans la revendication 14, dans laquelle lesdites nanoparticules de SMA de masse moléculaire élevées sont encapsulées dans des liposomes, et lesdites nanoparticules de SMA de masse moléculaire élevée encapsulées dans des liposomes se déplacent dans le canal déférent pour pénétrer la zone épithéliale du tissu prostatique.
